# EUROPÄISCHE PATENTSCHRIFT

(11) **EP 2 605 780 B1**
(45) Veröffentlichungstag und Bekanntmachung des Hinweises auf die Patenterteilung: **23.10.2019**
(21) Anmeldenummer: 11752152.6
(22) Anmeldetag: 17.08.2011
(51) Int. Cl.: A61K 33/06, A61K 33/10, A61K 9/00

(54) **KONZENTRAT**
CONCENTRATE FOR MEDICAL SOLUTIONS, PRODUCTION THEREOF AND USE THEREOF IN DIALYSIS
CONCENTRÉ POUR SOLUTIONS MÉDICALES, SA PRODUCTION ET SON UTILISATION DANS LA DIALYSE

(30) Priorität: 18.08.2010 DE 102010039489
(43) Veröffentlichungstag der Anmeldung: 26.06.2013
(73) Patentinhaber: Fresenius Medical Care Deutschland GmbH, 61352 Bad Homburg (DE)
(72) Erfinder: SCHWEITZER, Thomas, 66578 Schiffsweiler (DE); FICHERT, Thomas, 66606 Sankt Wendel (DE); MATHIS, Pascal, 66359 Bous (DE)
(74) Vertreter: Dörries, Hans Ulrich
(86) Internationale Anmeldenummer: PCT/EP2011/064180
(87) Internationale Veröffentlichungsnummer: WO 2012/022775

(56) Entgegenhaltungen:
- WO-A1-99/27885
- WO-A1-2011/073274
- DD-A1- 247 841
- DD-A1- 247 842
- DD-A1- 247 844
- US-A- 2 683 664
- US-A- 4 489 535
- Anonymous: "Vital Active Getränkekonzentrate", Energy Body Nàhrwertinfos , 2009, XP002660577, Gefunden im Internet: URL:http://www.body-fashion.de/Energy-Shop /contents/de/Vital_Active_de.pdf [gefunden am 2011-10-05]

## Beschreibung

Die vorliegende Erfindung betrifft verbesserte Konzentrate für medizinische Lösungen, deren Herstellung, als auch deren Verwendung in der Dialyse.

### Hintergrund

Gängigerweise werden Dialyselösungen in zentralen Anlagen in Behandlungszentren hergestellt und über ein Rohrsystem an die einzelnen Behandlungsstationen weitergeleitet. Alternativ werden in Behandlungszentren großvolumige Kanister verwendet, von denen die vorbereitete Dialyselösung an die Behandlungsstationen geleitet wird. Solche zentralen Versorgungseinrichtungen für Dialyselösungen sind problematisch im Hinblick auf deren Wartung und die Desinfizierung der gesamten Anlage. Obwohl diese Schwierigkeiten zuverlässig gehandhabt werden können, verursachen sie einen unerwünschten Aufwand.

Nachteilig an der zentralen Versorgung mit Dialyselösung ist auch die fehlende Individualisierung in der Herstellung, wodurch auf die Bedürfnisse der einzelnen Patienten durch eine individuelle Zusammensetzung der Dialyselösung während der Behandlung nicht eingegangen werden kann.

In der Herstellung von Dialyselösungen geht man daher immer mehr dazu über, diese vor Ort an der Behandlungsstation aus vorgelegten Konzentraten herzustellen. Das hat den Vorteil, mit wenig Aufwand aus einer geringen Menge an Konzentrat große Volumen behandlungsfertiger Lösung herstellen, und die Zusammensetzung der Lösung individuell steuern zu können. Notwendige zusätzliche Komponenten sind lediglich eine Wasserquelle und eine "reverse osmosis" (RO) Anlage an der Behandlungsstation oder in der Nähe der Behandlungsstation.

Die Verwendung der üblichen physiologisch verträglichen Säurekomponenten wie Salzsäure oder Essigsäure machte eine flüssige Konzentratform notwendig. Flüssige Konzentratformen können maschinell einfach dosiert werden, so dass die Einstellung von individuellen Lösungszusammensetzungen einfach vorgenommen werden kann. Insbesondere kann die Zusammensetzung auch während der Dialysebehandlungen variiert werden, was in einzelnen Fällen therapeutische Vorteile haben kann.

Nachteilig an den bekannten Lösungskonzentraten ist, dass sowohl für die Herstellung der flüssigen Form in der Produktion als auch für den Transport Ressourcen aufgewendet werden müssen, die bei festen Konzentratvorlagen nicht in Anspruch genommen werden müssten. Zum einen müssen die für Flüssigkonzentrate üblicherweise verwendeten Beutelsysteme bestimmte Eigenschaften aufweisen. Sie müssen zum Beispiel eine entsprechende Fallfestigkeit aufweisen, Lagerstabilität der Konzentrate gewährleisten, oder entsprechende Knickstabilität der Folie aufweisen. Zum anderen enthalten die Flüssigkonzentrate, die z.B. 125-fach konzentriert sind, eine hohe Konzentration an Säure, was zu einer hohen Acidität führt mit pH Werten im Bereich von pH 0 bis 1. Ein solches Konzentrat ist als Gefahrstoff zu beurteilen, der bei Unfällen, Leckage usw. besonders vorsichtig und fachgerecht gehandhabt werden muss.

Es gibt daher Bestrebungen, flüssige Konzentrate zu vermeiden, und das Konzentrat in fester Form vorzulegen. Eine Individualisierung der fertigen Dialyselösung ist dann allerdings nur mit einigem Aufwand zu erreichen. Es können verschiedene Trockenkonzentratvorlagen unterschiedlicher Zusammensetzung hergestellt werden, die für individuelle Anwendungen angepasst werden können. Insbesondere ist eine Variation der Kaliumbestandteile erwünscht, um Dialyselösungen mit unterschiedlicher Kaliumkonzentration bereitzustellen, um unterschiedlichen Patientenbedürfnissen gerecht zu werden.

Trockenkonzentrate sind vor allem zur Herstellung eines Dialyse-Batch interessant. Hierbei wird das gesamte Volumen an Dialyselösung in einem Lösungsprozess hergestellt und für die Dialyse bereitgestellt.

Die üblichen Komponenten für die Herstellung einer Dialyselösung sind Magnesiumchlorid, Kalziumchlorid, Natriumchlorid, Kaliumchlorid, Natriumhydrogenkarbonat, Glukose und eine physiologisch verträgliche Säure, z.B. Salzsäure, Essigsäure, oder Zitronensäure. Bei einer festen Formulierung kommen im Wesentlichen nur feste Säuren als Säurekomponente in Frage. Flüssige Säuren lösen im Allgemeinen das Konzentrat an und ergeben eine andere Darreichungsform, die z.B. auch in so genannten "Slurries" (Suspensionen mit hohem Feststoffanteil) Verwendung findet.

Die Kombination der Komponenten kann zu physikalisch / chemischen Unverträglichkeiten führen, was zur Verschlechterung des Auflöseverhaltens des Konzentrates führen kann, und die Lagerstabilität beeinträchtigt.

Zum Beispiel ist aus dem Stand der Technik bekannt, dass Glukose, die üblicherweise als Osmotikum eingesetzt wird, zusammen mit den anderen Komponenten wie Zitronensäure und Natriumhydrogenkarbonat nicht lagerstabil ist. Glukose besitzt bei relativ geringer Konzentration eine hohe Osmolarität und ist gut verträglich. Für den Einsatz von Glukose spricht insbesondere der Preisvorteil gegenüber anderen möglicherweise als Osmotikum einsetzbaren Stoffen.

Im Stand der Technik gibt es mehrere Vorschläge, um bei Trockenkonzentraten die Wechselwirkung zwischen Glukose und anderen Komponenten zu vermeiden.

EP 1 192 960 B1, EP 1 192 961 B1, JP 200823958, EP 1 086 700 B1, und EP 1 059 083 B1 beschreiben Trockenkonzentrate, bei denen die Granulate mehrschichtig aus den für die Dialyselösungsherstellung notwendigen Komponenten aufgebaut sind. Dabei werden Glukoseschichten, oder in einer Schicht umschlossene Bereiche von Glukose von den anderen Komponenten durch Pufferschichten getrennt, um eine chemische Wechselwirkung und/oder eine Degradation der Glukose zu vermeiden. Die Pufferschicht besteht z.B. aus Natriumchlorid.

Nachteilig an solchen Granulaten ist jedoch, dass Glukose mit Natriumchlorid in Kontakt ist, was nach längerer Lagerung zu "Caking" führen kann. Beim "Caking" handelt es sich um eine Agglomeratbildung einer an sich pulverförmigen Substanz, eines Granulates oder einer pelletförmigen oder tablettenförmigen Substanz. "Caking" entsteht, indem sich die Partikel durch partielle Lösungsvorgänge oder andere Transporterscheinungen verbinden und zusammenlagern. Das "Caking" wird durch Wasser- und Wärme-Einfluss gefördert.

Des Weiteren sind die verwendeten Elektrolytkomponenten Magnesiumchlorid und Kalziumchlorid hygroskopisch und neigen dazu, teilweise mit dem eigenen Hydratwasser sogenannte "Slurries" zu bilden. In "Slurries" liegen Feststoffe und gelöste Stoffe in einer flüssigen Phase nebeneinander vor. Der Feststoffanteil einer solchen Mischung ist so groß, dass das Erscheinungsbild durch eine breiartige oder pastöse Viskosität geprägt ist.

JP 3589489 B2 und EP 1 458 433 A1 schlagen zur stabilen Lagerung von Trockenkonzentraten vor, die für die Dialyselösung benötigten Komponenten mehrschichtig in einem Beutel anzuordnen, wobei Glukose mit einer Pufferschicht aus Natriumchlorid von einer weiteren, eventuell wechselwirkenden Schicht, z.B. Natriumhydrogenkarbonat, getrennt vorgelegt wird. Das Problem der "Slurry"-Bildung bedingt durch die Hygroskopie der Komponenten Magnesiumchlorid und Kalziumchlorid wird aber nicht angegangen, und auch die Problematik des "Cakings" zwischen Glukose und Natriumchlorid wird nicht gelöst.

US4489535 A offenbart Zusammensetzungen, umfassend eine Säure enthaltende hochkonzentrierte Calciumsalzlösung, die gegebenenfalls auch Magnesiumionen und geringe Mengen anderer physiologisch akzeptabler Metallionen enthalten kann; und ein teilchenförmiges Gemisch, das Natriumhydrogencarbonat, Natriumchlorid und gegebenenfalls Kaliumchlorid, Dextrose und andere physiologisch akzeptable Materialien umfasst, die in wässriger Lösung nicht mit Natriumhydrogencarbonat reagieren.

WO 99/27885 A1 offenbart einen Behälter für medizinische Lösung, insbesondere für die Peritonealdialyse. Ein großes Kompartiment enthält den Großteil der medizinischen Lösung wie Natriumhydrogencarbonat, Natriumlactat und Natriumchlorid. Außerdem sind mehrere kleine Kompartimente vorgesehen, die Teilmengen der medizinischen Lösung enthalten, die für eine Langzeitlagerung nicht mit dem Inhalt des großen Kompartiments kompatibel sind, wie z. B. Calciumionen und Glucose.

WO 2011/073274 A1 betrifft ein Verfahren zum Auflösen bzw. Mischen eines Konzentrats in oder mit einem Fluid in einem Mehrkammerbeutel und ein Verfahren zur Herstellung eines medizinischen Fluids, insbesondere eines Dialysefluids, in einem Mehrkammerbeutel.

DD 247844 A1 offenbart ein Verfahren zur Herstellung von acethaltigem Elektrolyt-Feststoffgemisch. Das Verfahren ist gekennzeichnet durch Verwendung von z. B. besonderer Mischweise, Einsatz von Erdalkalikarbonaten, bestimmter Siebfeinheiten der Elektrolytkomponenten u. a. Lösungselemente im Rahmen eines besonderen Verfahrensablaufes.

JP 2001340423 A2 schlägt vor, Glukose von anderen Komponenten getrennt zu lagern, um das "Caking" der Glukose, oder Degradation von Glukose durch Wechselwirkung mit anderen Komponenten zu vermeiden. Die Problematik der "Slurry"-Bildung bedingt durch die Elektrolytkomponenten Magnesiumchlorid und Kalziumchlorid bleibt aber bestehen.

Der vorliegenden Erfindung liegt die Aufgabe zugrunde, ein Konzentrat zur Verfügung zu stellen, das lagerstabil ist und eines oder mehrere der oben genannten Probleme vermeidet.

Diese Aufgabe wird erfindungsgemäß durch die Merkmale des Anspruchs 1 gelöst. Vorteilhafte Weiterbildungen dieses Aspekts der Erfindung sind in den abhängigen Ansprüchen 2-19 gekennzeichnet.

Ein Konzentrat gemäß der Erfindung ist eine im wässrigen Medium lösliche Zusammensetzung aus Komponenten, die dazu geeignet ist, nach Zusatz eines wässrigen Mediums als medizinische Lösung, und bevorzugt als Dialyselösung, zu dienen. Das erfindungsgemäße Konzentrat liegt in trockener Form, also als Trockenkonzentrat vor. Beschrieben sind auch Konzentrate in flüssiger, halbfester oder pastöser Form sofern die Stabilität des Konzentrates, die Verdünnung im Löseprozess, Wechselwirkungen mit dem Konzentratkontainer und Qualitätsprüfung nicht negativ beeinflusst werden. Im Hinblick auf die Dosierung und Verwendung von Trockenkonzentraten zur Herstellung von Konzentratbehältern, wie beispielsweise einem Mehrkammerbeutelsystem, sind rieselfähige Konzentrate bevorzugt. Ein einheitlicher Wassergehalt für solche Trockenkonzentrate kann nicht angegeben werden. Dieser hängt von der genauen Zusammensetzung der Komponenten des Konzentrates ab. Jedoch sollte in keinem Fall der Wassergehalt so hoch liegen, dass ein Verklumpen ("Caking") vor oder während der Herstellung oder während der Lagerung des Konzentratbehälters eintritt. Wird als Konzentratbehälter ein Folienbeutel verwendet, wie er als bevorzugte Ausführung beschrieben ist, so sind rieselfähige Trockenkonzentrate die bevorzugte Wahl für den Herstellungsprozess wie auch für die Lagerung des Konzentratbehälters.

Ein typisches Konzentrat enthält mehre Komponenten, z.B. 2, 3, 4, 5, 6, 7, 8, 9, 10, 12 und bis zu 20 Komponenten, üblicherweise jedoch 7, 8 oder 9 Komponenten. Dabei bezeichnet der Begriff "Konzentrat" nicht unbedingt die Gesamtheit sämtlicher Komponenten, die in der schließlich zur Anwendung kommenden medizinischen Lösung enthalten sind. Vielmehr können dem Konzentrat auch noch Komponenten hinzugefügt werden, die gemeinsam mit dem Konzentrat und Wasser die gebrauchsfertige medizinische Lösung ergeben. Bevorzugt enthält das Konzentrat mindestens eine Komponente ausgewählt aus einer Pufferkomponente, einer Elektrolytkomponente und einer osmotischen Komponente. In einer besonders bevorzugten Ausführungsform enthält das Konzentrat mindestens eine Pufferkomponente, eine Elektrolytkomponente und eine osmotischen Komponente.

Die im Konzentrat enthaltenen Komponenten können alle üblichen Komponenten einer Dialyselösung sein, also Pufferkomponenten wie Natriumhydrogenkarbonat, Lactat, Pyruvat, Acetat, Zitrat, TRIS (Trishydroxymethylaminomethan), Aminosäuren oder Peptide oder andere dem Fachmann geläufige Pufferkomponenten; osmotische Komponenten wie Glukose, Glukosepolymere wie z.B Maltodextrin oder Icodextrin, Cyclodextrin, modifizierte Stärke, Polyole, Fructose, Aminosäuren, Peptide, Proteine, Aminozucker, Glycerin, N-Acetylglucosamin usw; Elektrolytkomponenten wie Natrium-, Kalium-, Kalzium-, oder Magnesiumchlorid usw. Des weiteren können physiologisch verträgliche Säuren enthalten sein, wobei sich die Verwendung von fester Zitronensäure etabliert hat und somit bevorzugt ist. Die Vorteile von Zitronensäure ergeben sich aus der einfachen Verfügbarkeit der Säure in der erforderlichen pharmazeutischer Reinheit. Darüber hinaus hat Zitronensäure antikoagulierende Wirkung, so dass in den Blutkontaktzonen, z.B. innerhalb des Dialysefilters, einer Koagulation vorgebeugt werden kann. Gegebenenfalls können auch noch weitere Komponenten enthalten sein. Die verwendeten Komponenten sollten vorzugsweise den Qualitätsanforderungen des Arzneibuchs, (z.B. Ph. Eur.) entsprechen. Die genaue Zusammensetzung und quantitativen Verhältnisse können je nach Anwendungsfall unterschiedlich sein.

Das wässrige Medium zur Herstellung der medizinischen Lösung aus dem Konzentrat ist üblicherweise Wasser, und bevorzugt RO-(reverse osmosis) Wasser. Jedes andere dem Fachmann geläufige geeignete wässrige Medium kann aber auch verwendet werden, zum Beispiel ein Destillat, oder eine Teillösung, die in Kombination mit dem erfindungsgemäßen Konzentrat die fertige medizinische Lösung, vorzugsweise Dialyselösung, ergibt.

Die Konzentratkomponenten gemäß der Erfindung können gemeinsam vorgelegt werden. Bevorzugt sollten die verschiedenen Komponenten jedoch auf mehrere Einzelkompartimente verteilt werden, z.B. auf 2 oder mehrere Einzelkompartimente, bevorzugt auf 3, 4 oder mehr Einzelkompartimente. Unter "Kompartiment" wird eine räumliche Trennung der Komponenten verstanden. In einer besonders bevorzugten Ausführungsform werden solche Kompartimente durch einen in mehrere Kammern getrennten Beutel (Mehrkammerbeutel) gebildet, bei dem die Kompartimente vorzugsweise durch Peelnähte (Siegelverbindungen zwischen Folien, die getrennt werden können, ohne dass ein Folienbruch oder eine Delamination der meist mehrschichtig aufgebauten Folien erfolgt) voneinander getrennt sind.

Bei Befüllen des Mehrkammerbeutels mit Wasser werden die Peelnähte getrennt, die Kompartimente setzen die Komponenten frei und werden in dem einströmenden Wasser gelöst. Das Prinzip dieses Lösungsverfahrens ist bereits in der WO 2007/144427 A2 und in der JP 7299134 A2 beschrieben. Für den Lösungsvorgang des Konzentrates sowohl aus einem einzigen Vorratsbehältnis, als auch aus einem Mehrkammerbeutelsystem ist es wichtig, dass die Konzentratkomponenten in einer trockenen und mit Wasser schnell durchmischbaren Form vorliegen. Eventuell gebildete "Caking"-Produkte verhindern dabei ein Lösen der Komponenten in einem angemessenen Zeitraum. Zum anderen kann die Bildung von "Slurries" dazu führen, dass Bestandteile (z.B. PVC, PET, Weichmacher, Kleberschicht) der Beutelfolie in die "Slurry"-Masse eluieren und das Konzentrat kontaminieren. Auch ist eine einfache optische Prüfung der Unversehrtheit eines Konzentratkompartiments, z.B. optische Prüfung auf Risse in dem Folienmaterial durch scharfe Granulate, bei Vorlage eines "Slurry" nicht mehr einfach möglich.

Wenn die Konzentratkomponenten in mehreren Einzelkompartimenten verteilt vorliegen sollen, ist es notwendig, verschiedene Komponenten miteinander zu kombinieren. Die Kombination aus hygroskopischen und nicht hygroskopischen, sowie sauren und basischen Komponenten stellt dabei die größte Herausforderung dar. Verschiedene Experimente zur Kombination der einzelnen Komponenten resultierten zunächst in dem folgenden Schema:

| | |
|---|---|
| Elektrolyte-Kompartiment "A": | Natrium-, **Kalium**-, Kalzium-, Magnesiumchlorid, Zitronensäure |
| Glukose-Kompartiment "B": | Glukose |
| Bikarbonat-Kompartiment "C": | Natriumchlorid, Natriumhydrogenkarbonat |

Es wurde jedoch festgestellt, dass aus der oben aufgeführten Aufteilung der Komponenten in pulverförmiger oder granulierter Form auf drei oder mehrere Kompartimente weitere Probleme resultierten, die durch die vorliegende Erfindung gelöst wurden.

Zum einen wurde festgestellt, dass bedingt durch die Hygroskopie der eingesetzten Elektrolyt-Komponenten Magnesiumchlorid (MgCl₂ x 6H₂O) und Kalziumchlorid (CaCl₂ x 2H₂O) eine nicht unerhebliche Wasseraufnahme im Elektrolyte-Kompartiment "A" auftritt. Dieser Effekt ist einerseits auf die Wasseraufnahme von außen durch das Primärpackmittel hindurch, als auch auf das Lösen der Salze im eigenen Kristallwasser zurückzuführen.

Diese Wasseraufnahme ist sehr problematisch, da sie zu einer physikalisch-chemischen Veränderung der Komponenten führt, was verschiedene Auswirkungen zur Folge hat. Zum einen ändert sich das Auflöseverhalten der Komponenten durch die Veränderung vom festen in den halbfesten Aggregatzustand. Des weiteren kann es zu Interaktionen zwischen dem Füllgut und dem Primärpackmittel kommen. Feuchte Füllgüter können zudem die Migration von Bestandteilen des Verpackungsmaterials in das Füllgut bewirken. Des Weiteren kann es zu Folienverletzungen des Primärpackmittels durch Veränderungen der Granulatgrößen, z.B. durch Kristallwachstum, kommen. Bei der Verwendung von Mehrkammerbeuteln kann sich ein weiteres Problem dadurch ergeben, dass Flüssigkeit aus dem Elektrolyte-Kompartiment "A", durch das Verpackungsmaterial hindurch in andere Kompartimente übertritt, was wiederum zu physikalischen-chemischen Veränderungen der Komponenten in den anderen Kompartimenten führen kann. So könnte zum Beispiel das Eindringen von Feuchtigkeit in das Bikarbonat-Kompartiment "C" zur Reaktion von Natriumhydrogen-karbonat zu Natriumkarbonat führen. Die damit verbundene Freisetzung von CO₂ kann den pH-Wert der finalen Lösung negativ beeinflussen.

Ein weiteres Problem liegt in der Veränderung des optischen Erscheinungsbildes der Komponenten, da unter Umständen die Komponenten durch die oben angesprochenen Veränderungen nicht mehr in allen Kompartimenten in trockenem und festem Zustand vorliegen. Dies führt zu einer Verunsicherung des Anwenders, da eine Entscheidung darüber, ob das Produkt fehlerhaft ist oder nicht, nicht ohne weiteres getroffen werden kann (z.B., die Komponenten eines von mehreren Kompartimenten sind halbfest).

Die vorliegende Erfindung löst das Problem der Wasseraufnahme durch die hygroskopischen Komponenten durch das Bereitstellen eines Konzentrats, in welchem die Komponente Magnesiumchlorid (MgCl₂ x 6H₂O) durch Magnesiumkarbonat ersetzt wird.

Überraschend wurde nämlich festgestellt, dass die Verwendung von Magnesiumkarbonat anstelle von Magnesiumchlorid zu verminderter Hygroskopie und höherer Stabilität des Konzentrats führt. Dabei wird die Bildung eines "Slurry" verhindert. Das führt zu einem verbesserten Auflöseverhalten und einer besseren Dosierfähigkeit des Konzentrates. Im weiteren wird dadurch die Übertragung von Feuchtigkeit auf andere Komponenten des Konzentrates, auch auf solche, die in anderen Kompartimenten vorliegen, verhindert. Dadurch bleiben diese Komponenten chemisch und physikalisch unverändert, es treten keine der oben genannten chemischen Reaktionen auf. Auch die Migration von Bestandteilen des Primärpackmittels in das Füllgut wird dadurch verhindert. In einer bevorzugten Ausführungsform wird basisches Magnesiumkarbonat (4MgCO₃ x Mg(OH)₂ x 5H₂O) verwendet.

In einem weiteren Aspekt der Erfindung wird ein Konzentrat bereitgestellt, in dem nicht nur Magnesiumchlorid (MgCl₂ x 6H₂O) durch Magnesiumkarbonat, sondern auch Kalziumchlorid (CaCl₂ x 2H₂O) ersetzt wird. Anstelle von dem üblicherweise verwendeten Kalziumchlorid mit der Formel CaCl₂ x 2H₂O wird gemäß der vorliegenden Erfindung wasserfreies Kalziumchlorid (CaCl₂) eingesetzt. Der Einsatz von wasserfreiem Kalziumchlorid verhindert das Lösen im eigenen Kristallwasser oberhalb von etwa 30°C. Durch die Kombination von basischem Magnesiumkarbonat und wasserfreiem Kalziumchlorid kann die Wasseraufnahme in das Trockenkonzentrat besonders effektiv unterbunden werden.

Bevorzugt wird das erfindungsgemäße Konzentrat in einem Mehrkammerbeutelsystem zur Verfügung gestellt. Wird das erfindungsgemäße Konzentrat in einem solches Mehrkammerbeutel verwendet, werden die Komponenten Magnesiumkarbonat, bevorzugt basisches Magnesiumkarbonat, und Kalziumchlorid, bevorzugt in wasserfreier Form, gemeinsam in einem Elektrolyte-Kompartiment "A" vorgelegt, das im wesentlichen frei von Magnesiumchlorid ist, d.h., die Anteile von MgCl₂ sollten kleiner 5% in Gewichtsprozent der eingesetzten Magnesiumsalze sein, bevorzug kleiner als 4%, 3%, 2%, 1%, 0.5%, oder 0.1%.

Das Elektrolyte-Kompartiment "A" ist, enthält neben den beiden Komponenten Magnesiumkarbonat und Kalziumchlorid bevorzugt auch noch ein oder mehrere physiologisch verträglichen Säure, wie z.B. Zitronensäure, und/oder andere, dem Fachmann bekannte, feste, physiologisch verträgliche Säuren, wie z.B. Äpfelsäure, Fumarsäure, Isozitronensäure, Bernsteinsäure, oder Oxalsäure. Des weiteren kann Kaliumchlorid enthalten sein.

Ein weiteres Problem ist das Zusammenbacken ("Caking") der Komponenten. Das "Caking" der Komponenten kann zu Auflösungsverzögerungen, Inhomogenität oder Partikelbildung der Komponenten führen. Bei Beutelsystemen kann das "Caking" außerdem zu Folienverletzungen führen, die in Leckagen des Gesamtsystems resultieren können.

Es wurde nun gefunden, dass das "Caking" der Komponenten dadurch verbessert und sogar unterbunden werden kann, indem Glukose in Form von wasserfreier Glukose verwendet wird, oder, indem Glukose einzeln, von den anderen Komponenten räumlich getrennt, vorgelegt wird, beispielsweise in einem Kompartiment "B". In einer bevorzugten Ausführungsform wird Glukose von den anderen Komponenten räumlich getrennt, beispielsweise in einem Kompartiment "B", vorgelegt, und in Form von wasserfreier Glukose verwendet.

Gemäß einem weiteren Aspekt der Erfindung werden die beiden Komponenten Natriumchlorid und Natriumhydrogenkarbonat von allen anderen Komponenten getrennt vorgelegt, zum Beispiel in einem Bikarbonat-Kompartiment "C", das nur diese beiden Salze und keine weiteren Komponenten enthält. Dadurch kann das "Caking" in diesem Kompartiment verhindert und das Auflöseverhalten und die Lagerstabilität des Konzentrats noch weiter verbessert werden.

Ein beispielhaftes erfindungsgemäßes Konzentrat ist schematisch in folgender Tabelle dargestellt:

| | |
|---|---|
| Elektrolyte-Kompartiment "A": | Magnesiumkarbonat; Kalziumchlorid (bevorzugt wasserfrei), Kaliumchlorid, physiologisch verträgliche Säure, z.B., Zitronensäure |
| Glukose-Kompartiment "B": | Glukose (bevorzugt wasserfrei) |
| Bikarbonat-Kompartiment "C": | Natriumchlorid, Natriumhydrogenkarbonat |

Die einzelnen Komponenten des Konzentrats gemäß der Erfindung können in den unten aufgeführten Mengenverhältnissen vorhanden sein. Die Zusammensetzungsbereiche variieren ja nach gewählter Therapieform der extrakorporalen Blutbehandlung oder der Art und Verwendung der medizinischen Lösung. Die Zusammensetzungen der Konzentrate können unterschiedlich angepasst werden, um gebrauchfertige Lösungen zu erhalten, die im folgenden Zusammensetzungsbereich liegen:

| | |
|---|---|
| Ca²⁺: | 0-2 mmol/l, z.B. 1,0; 0,8; 1,2; 1,5; 1,7; 0,5; 0,1 oder 0,3- 1,7; 0,5-1,5; 0,8-1,3 mmol/l |
| K⁺: | 0-130 mmol/l z.B.: 1; 2; 3; 4; 1,5; 2,5; 3,5; 4,5 oder 0-5; 1-4; 1,5-3,5; 2-3 mmol/l |
| HCO₃⁻: | 5-40 mmol/l: z.B. 22; 24; 25; 26; 27; 28; 29; 30; 31; 32; 33; 34; 35; 36; 37; 38; 39 oder 22-38; 24-36; 25-33; 28- 34; 30-37 mmol/l |
| Na⁺: | 10-150 mmol/l; z.B. 113; 118; 123; 125; 125,5; 126; 128; 130; 132; 134; 138; 140; 145; 148; 115-130; 120-128; 124-128; 120-135; 125-135; 130-140 mmol/l |
| Mg^{2+:} | 0-5 mmol/l; 0,1; 0,3; 0,6; 0,8 oder 0,1-0,8; 0,3-0,7; 0,5- 0,75 mmol/l |
| Cl⁻: | 10- 60 mmol/l, 100-140 mmol/l |
| Zitronensäure: | 0-5 mmol/l; z.B. 1; 2; 3; 4; 5 oder 0,8- 1,5 0,3- 2; 0,4- 4; 0,8- 3; 1-2,5 mmol/l |
| Glukose: | 0- 250 mmol/l; z.B. 5,55; 83 mmol/l oder 0 - 10; 60- 100 mmol/l |
| pH Wert: | pH = 6,8 - 7,8; bevorzugt pH = 7-7,6; am meisten bevorzugt pH = 7,3 oder pH = 7,4. |

Die physiologisch verträgliche Säure liegt üblicherweise in einem Überschuss von 0.5 mmol (bezogen auf die ursprüngliche Einwaage von Magnesiumkarbonat) vor. Die genauen Mengen der Komponenten des Konzentrats können vom Fachmann anhand seines allgemeinen Fachwissens und unter Berücksichtigung der jeweiligen Patientendaten, oder des jeweiligen Verwendungszwecks so gewählt werden, dass daraus die gewünschten medizinischen Lösungen resultieren.

Der Einsatz von basischem Magnesiumkarbonat (4MgCO₃ x Mg(OH)₂ x 4H₂O) in einem erfindungsgemäßen Konzentrat zur Herstellung einer medizinischen Lösung setzt eine gewisse Lösungsprozedur voraus, da das basische Magnesiumkarbonat nur im sauren Bereich gut löslich ist. Eine ausreichende Löslichkeit für den Lösungsprozess ist zum Beispiel bei einem pH Wert von pH≤4, wie zum BeispielpH=3 gegeben. Generell sollte der Lösungsprozess nicht über einem pH Wert von pH=4 vorgenommen werden, um eventuell ungelöste Partikel zu vermeiden. Gemäß einer Ausführungsform der vorliegenden Erfindung wird das trockene basische Magnesiumkarbonat zusammen mit einer physiologisch verträglichen festen Säure vorgelegt. Beim Zuleiten von Wasser, oder eines wässrigen Mediums, das sich zur Herstellung der medizinischen Lösung eignet, entsteht ein ausreichend saurer pH-Wert, so dass das basische Magnesiumkarbonat nach folgendem Reaktionsschema gelöst wird und eine "saure Grundlösung" entsteht:

4MgCO₃ x 2Mg(OH)₂ x 5H₂O + 12H⁺ → 6Mg²⁺ + 4CO₂ + 13H₂O

Im Falle der Herstellung einer Dialyselösung oder einer Substitutionslösung mit Hilfe eines Mehrkammerbeutelsystems wird vorzugsweise in einem folgenden Schritt die Glukose aus dem Glukosekompartiment "B" entweder in gelöster oder teilweise gelöster (angelöster) Form oder direkt als trockenes Konzentrat der bereits vorhandenen sauren Grundlösung beigefügt. Alternativ können zur Lösungsherstellung die trockenen Komponenten der Kompartimente "A" (Elektrolytkompartiment) und "B" (Glukosekompartiment) zunächst vereint werden und mit einem wässrigen Verdünnungsmedium gelöst werden.

In einem weiteren Schritt werden ein oder mehrere Pufferkomponenten, oder das ein oder mehrere Pufferkomponenten enthaltende Konzentrat des Kompartiments "C" (Bikarbonatkompartiment) angelöst, oder direkt zu der bereits vorhandenen "sauren Grundlösung", dh., zur sauren Lösung von Magnesiumkarbonat, oder zur sauren Mischung des Konzentrates des Elektrolytekompartiments "A" umfassend Magnesiumkarbonat und eine physiologisch verträgliche Säure und Verdünnungsmittel, oder zu einer Mischung der Konzentrate aus den Kompartimenten "A" und "B" und Verdünnungsmittel zugegeben, so dass ein pH Wert von pH > 4 entsteht, bevorzug ein pH Wert zwischen pH=6 und pH=8, und besonders bevorzugt zwischen pH ≥ 6,8 und pH ≤ 7,8. Die Komponenten der vorgelegten "sauren Grundlösung" müssen dabei nicht zwangsläufig vollständig gelöst sein. Vorzugsweise ist das basische Magnesiumkarbonat bereits gelöst, wenn das Konzentrat aus dem Kompartiment "C" zugegeben wird. Dabei kommt es zur CO₂ Entwicklung, da das Bikarbonat Salz im sauren pH Wert Bereich gelöst wird:

NaHCO₃ + H⁺ → Na⁺ + H₂O + CO₂

Die CO₂ Entwicklung muss durch eine entsprechende Vorrichtung an der Lösungseinrichtung berücksichtigt werden. Vorzugsweise wählt man ein Belüftungssystem, bei dem überschüssiges CO₂ entweichen kann.

Das erfindungsgemäße Konzentrat eignet sich z.B. zur Herstellung von medizinischen Lösungen. Medizinische Lösungen sind vorzugsweise Dialyselösungen, z.B. eine Hämodialyselösung oder eine Peritonealdialyselösung; oder Substitutionslösungen, z.B. Hämofiltrationslösungen.

Anhand der Abbildungen und der unten aufgeführten Beispiele wird die Erfindung nachstehend eingehend erläutert.

### Beispiel 1:

Beispielhafte Konzentrat-Zusammensetzungen gemäß der vorliegenden Erfindung sind folgende:

| | Substanzeinwaage [g] | | | | | | |
|---|---|---|---|---|---|---|---|
| | Kompartiment A | | | | Kompartiment B | Kompartiment C | |
| | MgCO₃ | CaCl₂ | KCl | Zitronensäure | D-Glucose wasserfrei | Natriumchlorid | NaHCO₃ |
| Variante 1 | 3,01 | 8,62 | 0 | 11,97 | 62,00 | 391,22 | 166,78 |
| Variante 2 | 3,01 | 8,62 | 9,24 | 11,97 | 62,00 | 391,22 | 166,78 |
| Variante 3 | 3,01 | 8,62 | 18,50 | 11,97 | 62,00 | 391,22 | 166,78 |
| Variante 4 | 4,51 | 0 | 0 | 11,97 | 62,00 | 422,06 | 93,62 |
| Variante 5 | 4,51 | 0 | 9,24 | 11,97 | 62,00 | 422,06 | 93,62 |
| Variante 6 | 4,51 | 0 | 18,50 | 11,97 | 62,00 | 422,06 | 93,62 |

| | Konzentratzusammensetzung [%] | | | | | | |
|---|---|---|---|---|---|---|---|
| | Kompartiment A | | | | Kompartiment B | Kompartiment C | |
| | MgCO₃ | CaCl₂ | KCl | Zitronensäure | D-Glucose wasserfrei | Natriumchlorid | NaHCO3 |
| Variante 1 | 12,8 | 36,5 | 0,0 | 50,7 | 100,0 | 70,1 | 29,9 |
| Variante 2 | 9,2 | 26,2 | 28,1 | 36,4 | 100,0 | 70,1 | 29,9 |
| Variante 3 | 7,1 | 20,5 | 43,9 | 28,4 | 100,0 | 70,1 | 29,9 |
| Variante 4 | 27,4 | 0,0 | 0,0 | 72,6 | 100,0 | 82,5 | 17,5 |
| Variante 5 | 17,5 | 0,0 | 35,9 | 46,5 | 100,0 | 82,5 | 17,5 |
| Variante 6 | 12,9 | 0,0 | 52,9 | 34,2 | 100,0 | 82,5 | 17,5 |

| | Resultierende Konzentrationen [mmol/L] | | | | | | |
|---|---|---|---|---|---|---|---|
| | Kompartiment A | | | | Kompartiment B | Kompartiment C | |
| | MgCO₃ | CaCl₂ | KCl | Zitronensäure | D-Glucose wasserfrei | Natriumchlorid | NaHCO₃ |
| Variante 1 | 0,50 | 1,25 | 0,0 | 1,0 | 5,55 | 140,0 | 32,0 |
| Variante 2 | 0,50 | 1,25 | 2,0 | 1,0 | 5,55 | 140,0 | 32,0 |
| Variante 3 | 0,50 | 1,25 | 4,0 | 1,0 | 5,55 | 140,0 | 32,0 |
| Variante 4 | 0,75 | 0,0 | 0,0 | 1,0 | 5,55 | 140,0 | 18,0 |
| Variante 5 | 0,75 | 0,0 | 2,0 | 1,0 | 5,55 | 140,0 | 18,0 |
| Variante 6 | 0,75 | 0,0 | 4,0 | 1,0 | 5,55 | 140,0 | 18,0 |

Zusammensetzung und Konzentrationen eines typischen Batch-Bags vor der Umstellung auf MgCO₃ bzw. auf wasserfreie Substanzen (Vergleichsbeispiel):

| | Substanzeinwaage [g] | | | | | | |
|---|---|---|---|---|---|---|---|
| | Kompartiment A | | | | Kompartiment B | Kompartiment C | |
| | MgCl₂ x 6H₂O | CaCl₂ x H₂O | KCl | Zitronensäure | D-Glucose x H₂O | Natriumchlorid | NaHCO₃ |
| Vergleichsbeispiel | 6,32 | 13,64 | 9,24 | 17,36 | 62,00 | 375,1 | 190,34 |

| | Konzentratzusammensetzung [%] | | | | | | |
|---|---|---|---|---|---|---|---|
| | Kompartiment A | | | | Kompartiment B | Kompartiment C | |
| | MgCl₂ x 6H₂O | CaCl₂ x H₂O | KCl | Zitronensäure | D-Glucose x H₂O | Natriumchlorid | NaHCO₃ |
| Vergleichsbeispiel | 13,57 | 29,29 | 19,85 | 37,29 | 100 | 66,34 | 33,66 |

| | Resultierende Konzentrationen [mmol/L] | | | | | | |
|---|---|---|---|---|---|---|---|
| | Kompartiment A | | | | Kompartiment B | Kompartiment C | |
| | MgCl₂ x 6H₂O | CaCl₂ x H₂O | KCl | Zitronensäure | D-Glucose x H₂O | Natriumchlorid | NaHCO₃ |
| Vergleichsbeispiel | 13,57 | 29,29 | 19,85 | 37,29 | 5,55 | 103,50 | 36,50 |

Die erfindungsgemäßen Konzentrate liegen bevorzugt in einem oben beschriebenen Mehrkammerbeutelsystem vor. Es wurde festgestellt, dass die oben aufgeführten Konzentrate gemäß der Erfindung keine Wasseraufnahme, kein "Slurring" und kein "Caking" mehr zeigten. Stabilitätsuntersuchungen der neuen Konzentratzusammensetzungen zeigen eine homogene Lösung mit ausgezeichneter Stabilität.

### Beispiel 2

Die Wasseraufnahme in ein Elektrolyte-Kompartiment "A" unter Einsatz zweier Konzentratbeutel, jeweils hergestellt aus einer Gasbarrierefolie und aus gasdurchlässigem Foliematerial, wurde evaluiert. Das Konzentrat bestand dabei aus den herkömmlichen Bestandteilen:
- Natriumchlorid
- Magnesiumchlorid mit Kristallwasser, MgCl₂ x 6H₂O
- Kalziumchlorid mit Kristallwasser, CaCl₂ x 2H₂O
- Kaliumchlorid, KCl
- Zitronensäure, C₆H₈O₇

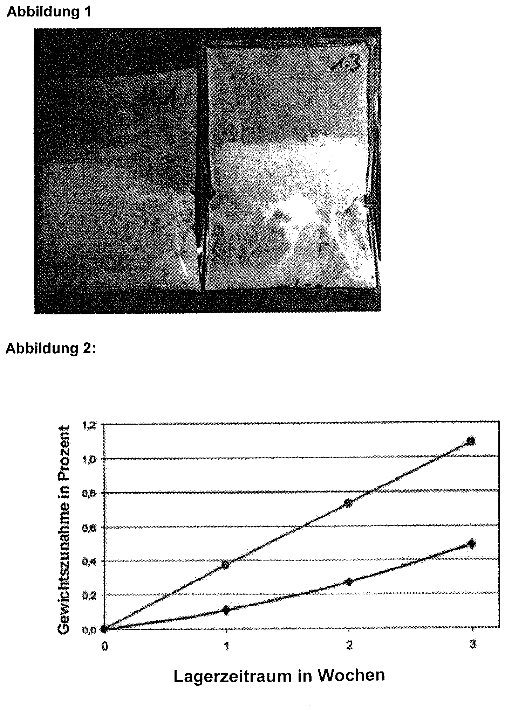

Die hermetisch verschweissten Beutel wurden bei 40°C und 75% relativer Luftfeuchtigkeit in einer Klimakammer gelagert und in regelmäßigen Abständen gewogen. Eine Zunahme des Gewichtes des Beutels ist auf eine Permeabilität für Wasserdampf und die damit verbundene Aufnahme von Wasser in das Konzentrat zurückzuführen. Wie erwartet führte die gaspermeable Folie zu einer schnelleren und auch größeren Gewichtserhöhung des Konzentratbeutels als die Folie mit Gasbarriere (siehe auch Abbildung 1, worin die Gewichtszunahme der Konzentratbeutel mit Gasbarriefolie (◆) bzw. mit gasdurchlässigem Folienmaterial (●) des Konzentrates enthaltend Natriumchlorid, KCl, CaCl₂, MgCl₂, Zitronensäure bei 40°C und 75 relativer Luftfeuchtigkeit dargestellt ist.)

Bei der gaspermeablen Folie wurden zur Herstellung die Rohstoffe:
- PolyPropylen -PP-
- PolyEthylen -PE-
- StyrolEthylenButylenStyrol Block Copolmyer -SEBS-
verwendet. Die gasimpermeable Folie wies zusätzliche eine keramische Barriereschicht aus Siliziumoxid auf.

Die Wasseraufnahme in Konzentratbeutel mit Gasbarrierefolie bzw. mit gasdurchlässigem Folienmaterial wurde des weiteren für Konzentrate bestehend aus Glukose x H₂O / Natriumchlorid bzw. NaHCO₃/ Natriumchlorid unter den gleichen Bedingungen getestet.

Die Glukose x H₂O/ Natriumchlorid Konzentratbeutel zeigten "Caking"- Verhalten.

Bei dem NaHCO₃ / Natriumchlorid Konzentrat wurde keine signifikante Wasseraufnahme festgestellt.

Dieses Ergebnis zeigt, dass die Wasseraufnahme zwar durch Einsatz einer Barrierefolie verringert, aber nicht vollständig verhindert werden konnte. Durch die erfindungsgemäß getrennte Vorlage von NaHCO₃ / Natriumchlorid in ein Kompartiment ohne andere Bestandteile kann die Wasseraufnahme und "Caking" verhindert werden.

### Beispiel 3:

Die Wasseraufnahme und das "Caking"-Verhalten eines herkömmlichen Trockenkonzentrates wurde anhand von zwei Konzentratbeuteln, mit bzw. ohne Gasbarriere, mit folgenden Konzentratkomponenten untersucht
- Glukose mit Kristallwasser, C₆H₁₂O₆ x H₂O
- Magnesiumchlorid mit Kristallwasser, MgCl₂ x 6H₂O
- Kalziumchlorid mit Kristallwasser, CaCl₂ x 2H₂O
- Kaliumchlorid, KCl
- Zitronensäure, C₆H₈O₇

Der Beutel aus Folienmaterial ohne nennenswerte Gasbarriereigenschaften ist in Abbildung 2 links abgebildet. Es wurden PolyPropylen -PP-, PolyEthylen -PE, StyrolEthylenButylenStyrol Block Copolmyer -SEBS-, für die Herstellung dieser Folie verwendet. Der in der Abbildung 2 rechts gezeigte Beutel bestand aus den gleichen Materialien. Zusätzlich enthielt diese Folie eine Gasbarriereschicht aus Siliziumoxid. Die Gaspermeabilität der Folie lag für CO₂ unter 20 cm³/(m²·d·bar) gemessen nach DIN 53380 - Teil 4. Die Beutel wurden in einer Klimakammer bei 40°C und einer relativen Luftfeuchtigkeit von 75% für zwei Wochen gelagert. In beiden Fällen wurde "Caking" beobachtet, zusätzlich trat eine braune Verfärbung ein, die auf eine Degradation der Glukose schließen lässt. Das "Caking" der Komponenten in den zwei Konzentratbeuteln nach 2 Wochen unter den oben genannten Bedingungen ist in der Abbildung 1 gezeigt.

### Beispiel 4:

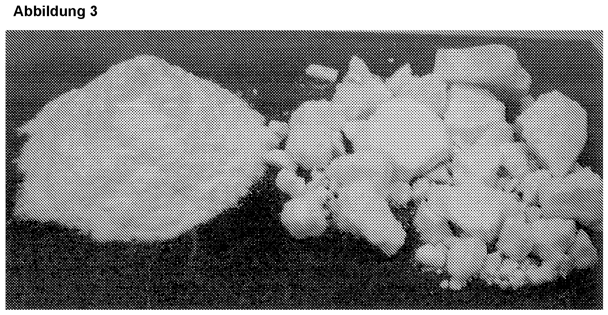

Die Stabilität eines Konzentrates mit erfindungsgemäßen Komponenten versus herkömmlichen Komponenten wurde getestet. Wie in Abbildung 3 gezeigt, trat bei einem herkömmlichen Konzentrat bestehend aus Glukose x H₂O / Natriumchlorid, das ursprünglich pulverförmig war (links in der Abbildung 3, T = 0 Monate) bei einer Bewitterung des Konzentratbeutels bei 40°C und 75% relativer Luftfeuchtigkeit in der Klimakammer "Caking" auf (dargestellt rechts in Abbildung 3, T= 6 Monate). Es bestand der Verdacht, dass das Kristallwasser der Glukose in Verbindung mit Natriumchlorid zum "Caking" führte. Natriumchlorid weist zudem eine gewisse Hygroskopie auf, so dass eine Wasserdampfpermeabilität durch die Folie nicht auszuschließen ist.

Dagegen zeigten weitere Untersuchungen, dass wasserfreie Glukose gemäß der Erfindung unter den gleichen Testbedingungen nicht zum "Caking" neigt, und sich damit besonders für eine mehrteilige trockene Konzentratzusammensetzung eignet.

### Beispiel 5:

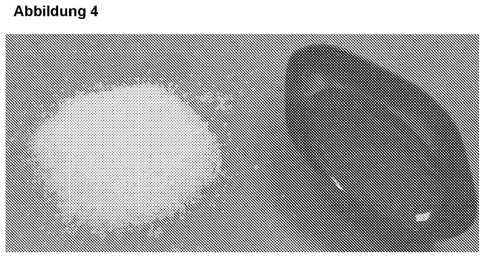

MgCl₂ x 6H₂O wurde in der Klimakammer bei 40°C und 75% relativer Luftfeuchtigkeit in einem gaspermeablen Konzentratbeutel gelagert. Das Magnesiumchlorid verflüssigte sich dabei durch Wasseraufnahme und Lösen im eigenen Kristallwasser (siehe auch Abbildung 4, links die zunächst pulverförmige MgCl₂ x 6H₂O Komponente zum Zeitpunkt T=0; rechts verflüssigtes MgCl₂ x 6H₂O nach 6 Monaten Bewitterung unter den oben genannten Bedingungen).

### Beispiel 6:

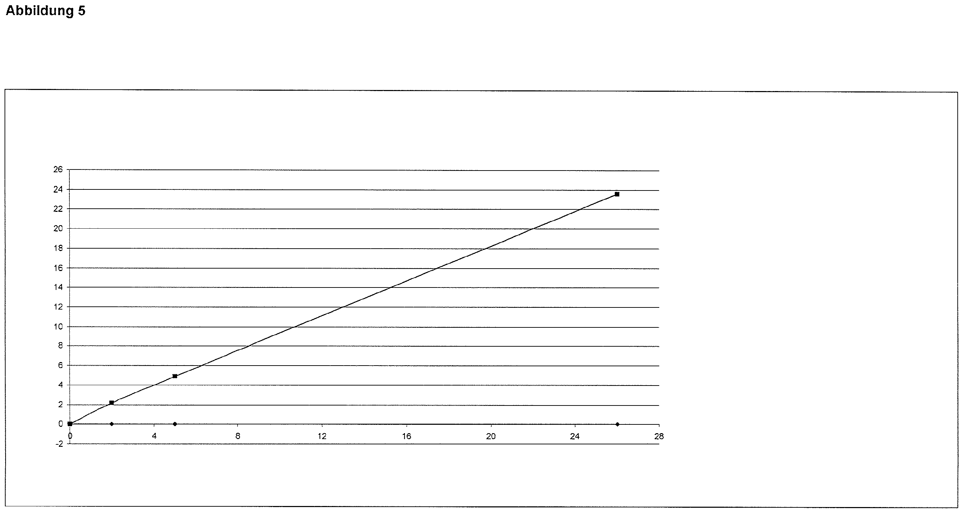

Die Wasseraufnahme in Konzentratbeutel ohne Gasbarriere, enthaltend MgCl₂ x 6H₂O bzw. basisches Magnesiumkarbonat 4 MgCO₃ x Mg(OH)₂ x 5H₂O, wurde verglichen. Dabei wurden die Konzentratbeutel jeweils in der Klimakammer bei 40°C und 75% relativer Luftfeuchtigkeit für mehrere Wochen bewittert. Das basische Magnesiumkarbonat nahm über einen Zeitraum von 6 Monaten kein Wasser auf, während bei Magnesiumchlorid eine signifikante Wasseraufnahme zu verzeichnen war. Siehe auch Abbildung 5: die graphische Darstellung der Wasseraufnahme in den Konzentratbeutel ohne Gasbarriere mit MgCl₂ x 6H₂O Inhalt ist mit (■) gekennzeichnet, und für 4 MgCO₃ x Mg(OH)₂ x 5H₂O mit (◆). Auf der Abszisse ist die Zeit in Wochen aufgetragen. Auf der Ordinate ist der Wassergehalt in Gewichtsprozent aufgetragen. Es erfolgt keine Wasseraufnahme in den mit basischem Magnesiumkarbonat gefüllten Beutel bei 40°C und 75% relativer Luftfeuchtigkeit, auch nicht nach ca. 26 Wochen, während nach dem gleichen Zeitraum die Wasseraufnahme in den mit MgCl₂ x 6H₂O befüllten Konzentratbeutel fast 24% betrug.

## Patentansprüche

1. Konzentrat zur Herstellung einer medizinischen Lösung, wobei das Konzentrat in trockener Form vorliegt, und wobei Komponenten des Konzentrats auf drei oder mehrere Kompartimente verteilt vorliegen, und wobei a) Magnesiumkarbonat und b) wasserfreies Kalziumchlorid gemeinsam in einem Kompartiment vorgelegt werden.

2. Konzentrat nach Anspruch 1, wobei
(a) das Konzentrat mindestens eine Elektrolytkomponente, mindestens eine osmotische Komponente und mindestens eine Pufferkomponente enthält; und/oder
(b) als Magnesiumkarbonat 4MgCO₃ x Mg(OH)₂ x 5H₂O verwendet wird; und/oder
(c) das Konzentrat wasserfreies Kalziumchlorid enthält; und/oder
(d) das Konzentrat eine physiologisch verträgliche Säure enthält, die bevorzugt aus der Gruppe bestehend aus Zitronensäure, Apfelsäure, Fumarsäure, Isozitronensäure, Bernsteinsäure und Oxalsäure ausgewählt ist; und/oder
(e) das Konzentrat Kaliumchlorid enthält; und/oder
(f) das Konzentrat Glukose enthält, wobei Glukose gegebenenfalls von den anderen Komponenten räumlich getrennt in einem Kompartiment vorliegt; und/oder
(g) das Konzentrat Natriumhydrogenkarbonat enthält, wobei Natriumhydrogenkarbonat gegebenenfalls von den anderen Komponenten räumlich getrennt in einem Kompartiment vorliegt; und/oder
(h) Komponenten des Konzentrats auf drei oder mehr Kompartimente verteilt vorliegen, wobei das erste Kompartiment Komponenten nach einem der Ansprüche 1 oder 2(a)-(d) enthält.

3. Konzentrat nach Anspruch 2(h), wobei das zweite Kompartiment Glukose enthält.

4. Konzentrat nach einem der Ansprüche 2(h) oder 3, wobei das dritte Kompartiment Natriumhydrogenkarbonat enthält.

5. Konzentrat nach einem der Ansprüche 1-4, wobei Komponenten des Konzentrats auf drei oder mehr räumlich getrennte Einzelkompartimente verteilt vorliegen, wobei das erste Kompartiment Komponenten nach einem der Ansprüche 1 oder 2(a)-(d) enthält, das zweite Kompartiment Glukose enthält, und das dritte Kompartiment Natriumhydrogenkarbonat enthält.

6. Konzentrat nach einem der Ansprüche 2(f)-(h) oder 3 bis 5, wobei wasserfreie Glukose verwendet wird.

7. Konzentrat nach einem der Ansprüche 2(g), 2(h) oder 3 bis 6, wobei Natriumchlorid zusammen mit dem Natriumhydrogenkarbonat von allen anderen Komponenten räumlich getrennt in einem Kompartiment vorliegt.

8. Konzentrat nach einem der Ansprüche 2(d)-(h) oder 3 bis 7, wobei die physiologisch verträgliche Säure Zitronensäure ist.

9. Konzentrat nach einem der Ansprüche 1 bis 8, wobei die Anteile von MgCl₂ kleiner als 5%, bevorzugt kleiner als 4%, 3%, 2%, 1 %, 0.5%, oder 0.1 % der eingesetzten Magnesiumsalze sind
oder das Konzentrat frei von Magnesiumchlorid ist.

10. Verwendung des Konzentrats nach einem der Ansprüche 1-9 zur Herstellung einer medizinischen Lösung.

11. Konzentrat nach einem der Ansprüche 1-9, oder Verwendung nach Anspruch 10, wobei die medizinische Lösung eine Dialyselösung oder eine Substitutionslösung ist.

12. Verwendung von Magnesiumkarbonat, vorzugsweise 4MgCO₃ x Mg(OH)₂ x 5H₂O, zur Herstellung eines Trockenkonzentrats für die Dialyse,
wobei das Konzentrat wasserfreies Kalziumchlorid enthält.

13. Mehrkammerbeutel, wobei der Mehrkammerbeutel das Konzentrat nach einem der Ansprüche 1-9 enthält.

14. Verfahren zur Herstellung einer medizinischen Lösung ausgehend von einem Konzentrat nach einem der Ansprüche 1-9, **dadurch gekennzeichnet, dass** in einem Schritt a) das Magnesiumkarbonat, oder der das Magnesiumkarbonat enthaltende Teil eines Konzentrates bei einem pH Wert von pH ≤ 4 gelöst wird, und
in einem Schritt b) zu der in a) erhaltenen Lösung eine oder die Pufferkomponente oder der die Pufferkomponente enthaltende Teil des Konzentrates hinzugefügt wird, so dass ein pH Wert von pH > 4 entsteht,
wobei die Pufferkomponente gegebenenfalls eine Komponente oder mehrere Komponenten umfassend Laktat, Acetat, Pyruvat, Natriumhydrogenkarbonat oder Citrat ist; und/oder
wobei der pH Wert in Schritt b) gegebenenfalls zwischen pH ≥ 6,8 und pH ≤ 7,8 liegt.

## Claims

1. A concentrate for producing a medical solution, wherein the concentrate is present in dry form, and wherein components of the concentrate are present distributed to three or more compartments, and wherein a) magnesium carbonate and b) anhydrous calcium chloride are provided together in one compartment.

2. The concentrate according to claim 1, wherein
(a) the concentrate contains at least one electrolyte component, at least one osmotic component and at least one buffer component; and/or
(b) is used as 4MgCO₃ x MG(OH)₂ x 5H₂O magnesium carbonate; and/or
(c) the concentrate contains anhydrous calcium chloride; and/or
(d) the concentrate contains a physiologically acceptable acid, preferably selected from the group consisting of citric acid, malic acid, fumaric acid, isocitric acid, succinic acid and oxalic acid; and/or
(e) the concentrate contains potassium chloride; and/or
(f) the concentrate contains glucose, wherein glucose is present if applicable spatially separated from the other components in a compartment; and/or
(g) the concentrate contains sodium bicarbonate, wherein sodium bicarbonate is present if applicable spatially separated from the other components in a compartment; and/or
(h) components of the concentrate are present distributed to three or more compartments, wherein the first compartment contains components according to one of claims 1 or 2(a)-(d).

3. The concentrate according to claim 2(h), wherein the second compartment contains glucose.

4. The concentrate according to one of claims 2(h) or 3, wherein the third compartment contains sodium bicarbonate.

5. The concentrate according to one of claims 1-4, wherein components of the concentrate are present distributed to three or more spatially separated individual compartments, wherein the first compartment contains components according to one of claims 1 or 2(a)-(d), the second compartment contains glucose, and the third compartment contains sodium bicarbonate.

6. The concentrate according to one of claims 2(f)-(h) or 3 to 5, wherein anhydrous glucose is used.

7. The concentrate according to one of claims 2(g), 2(h) or 3 to 6, wherein sodium chloride together with the sodium bicarbonate is present spatially separated from all other components in a compartment.

8. The concentrate according to one of claims 2(d)-(h) or 3 to 7, wherein the physiologically acceptable acid is citric acid.

9. The concentrate according to one of claims 1 to 8, wherein the proportions of MgCl₂ are less than 5%, preferably less than 4%, 3%, 2%, 1%, or 0.1% of the magnesium salts which are used
or the concentrate is free of magnesium chloride.

10. Use of the concentrate according to one of claims 1-9 for the production of a medical solution.

11. The concentrate according to one of claims 1-9, or the use according to claim 10, wherein the medical solution is a dialysis solution or a substitution solution.

12. Use of magnesium carbonate, preferably 4MgCO₃ x MG(OH)₂ x 5H₂O, for producing a dry concentrate for dialysis,
wherein the concentrate contains anhydrous calcium chloride.

13. A multi-chamber pouch, wherein the multi-chamber pouch contains the concentrate according to one of claims 1-9.

14. A method for producing a medical solution proceeding from a concentrate according to one of claims 1-9, **characterized in that** in a step a) the magnesium carbonate, or the part of a concentrate containing the magnesium carbonate is dissolved at a pH value of pH ≤ 4, and
in a step b) a or the buffer component or the part of the concentrate containing the buffer component is added to the solution obtained in a), so that a pH value of pH > 4 is produced,
wherein the buffer component if applicable is a component or several components comprising lactate, acetate, pyruvate, sodium bicarbonate or citrate; and/or
wherein the pH value in step b) if applicable lies between pH ≥ 6.8 and pH ≤ 7.8.

## Revendications

1. Concentré, destiné à préparer une solution médicale, le concentré se présentant sous forme sèche, et des composants du concentré se présentant en distribution sur trois ou plusieurs compartiments et a) du carbonate de magnésium et b) du chlorure de calcium anhydre étant présentés conjointement dans un compartiment.

2. Concentré selon la revendication 1,
(a) le concentré contenant au moins un composant électrolytique, au moins un composant osmotique et au moins un composant tampon ; et/ou
(b) en tant que carbonate de magnésium étant utilisé le 4MgCO₃ x Mg(OH)₂ x 5H₂O ; et/ou
(c) le concentré contenant du chlorure de calcium anhydre ; et/ou
(d) le concentré contenant un acide physiologiquement compatible, qui est sélectionné de préférence dans le groupe constitué de l'acide citrique, de l'acide malique, de l'acide fumarique, de l'acide isocitrique, de l'acide succinique et de l'acide oxalique ; et/ou
(e) le concentré contenant du chlorure de potassium ; et/ou
(f) le concentré contenant du glucose, le glucose se présentant le cas échéant physiquement séparé des autres composants dans un compartiment ; et/ou
(g) le concentré contenant du bicarbonate de sodium, le bicarbonate de sodium se présentant le cas échéant physiquement séparé des autres composants dans un compartiment ; et/ou
(h) des composants du concentré se présentant en distribution sur trois ou plusieurs compartiments, le premier compartiment contenant des composants selon l'une quelconque des revendications 1 ou 2(a) à (d).

3. Concentré selon la revendication 2(h), le deuxième compartiment contenant du glucose.

4. Concentré selon l'une quelconque des revendications 2(h) ou 3, le troisième compartiment contenant du bicarbonate de sodium.

5. Concentré selon l'une quelconque des revendications 1 à 4, des composants du concentré se présentant en distribution sur trois compartiments individuels physiquement séparés ou plus, le premier compartiment contenant des composants selon l'une quelconque des revendications 1 ou 2(a) à (d), le deuxième compartiment contenant du glucose et le troisième compartiment contenant du bicarbonate de sodium.

6. Concentré selon l'une quelconque des revendications 2(f) à (h) ou 3 à 5, du glucose anhydre étant utilisé.

7. Concentré selon l'une quelconque des revendications 2(g), 2(h) ou 3 à 6, du chlorure de sodium se présentant conjointement au bicarbonate de sodium, physiquement séparé de tous les autres composants dans un compartiment.

8. Concentré selon l'une quelconque des revendications 2(d) à (h) ou 3 à 7, l'acide physiologiquement compatible étant de l'acide citrique.

9. Concentré selon l'une quelconque des revendications 1 à 8, les parts de MgCl₂ étant inférieures à 5%, de préférence, inférieures à 4%, 3%, 2%, 1 %, 0.5%, ou 0.1 % des sels de magnésium mis en oeuvre,
ou le concentré étant exempt de chlorure de magnésium.

10. Utilisation du concentré selon l'une quelconque des revendications 1 à 9, pour la préparation d'une solution médicale.

11. Concentré selon l'une quelconque des revendications 1 à 9, ou utilisation selon la revendication 10, la solution médicale étant une solution de dialyse ou une solution de substitution.

12. Utilisation de carbonate de magnésium, de préférence de 4MgCO₃ x Mg(OH)₂ x 5H₂O, pour la préparation d'un concentré sec destiné à la dialyse,
le concentré contenant du chlorure de calcium anhydre.

13. Sachet à compartiments multiples, le sachet à compartiments multiples contenant le concentré selon l'une quelconque des revendications 1 à 9.

14. Procédé, destiné à préparer une solution médicale à partir d'un concentré selon l'une quelconque des revendications 1 à 9, **caractérisé en ce que** dans une étape a), on fait dissoudre le carbonate de magnésium, ou la partie d'un concentré contenant le carbonate de magnésium à une valeur pH de pH ≤ 4, et
dans une étape b), on ajoute à la solution obtenue en a) un ou le composant tampon ou la partie du concentré contenant le composant tampon, de sorte à obtenir une valeur pH de pH > 4,
le composant tampon étant le cas échéant un composant ou plusieurs composants comprenant le lactate, l'acétate, le pyruvate, le bicarbonate de sodium ou le citrate ; et/ou
le cas échéant, dans l'étape b), la valeur pH se situant entre pH ≥ 6,8 et pH ≤ 7,8.
